# EUROPÄISCHE PATENTSCHRIFT

(11) **EP 1 064 093 B1**
(45) Veröffentlichungstag und Bekanntmachung des Hinweises auf die Patenterteilung: **04.06.2003**
(21) Anmeldenummer: 99911776.5
(22) Anmeldetag: 11.03.1999
(51) Int. Cl.: B01J 31/18, C07C 45/50, C07F 9/6571

(54) **KATALYSATOR, UMFASSEND EINEN RHODIUM-KOMPLEX AUF DER BASIS EINES PHOSPHONITLIGANDEN UND VERFAHREN ZUR HYDROFORMYLIERUNG**
CATALYST COMPRISING A RHODIUM COMPLEX BASED ON A A PHOSPHONITE LIGAND AND METHOD FOR HYDROFORMYLATION
CATALYSEUR COMPRENANT UN COMPLEXE DE RHODIUM ASSOCIE A UN LIGAND PHOSPHONITE, ET PROCEDE D'HYDROFORMYLATION

(30) Priorität: 12.03.1998 DE 19810794
(43) Veröffentlichungstag der Anmeldung: 03.01.2001
(73) Patentinhaber: BASF AKTIENGESELLSCHAFT, 67056 Ludwigshafen (DE)
(72) Erfinder: MAAS, Heiko, D-67105 Schifferstadt (DE); PACIELLO, Rocco, D-67098 Bad Dürkheim (DE); RÖPER, Michael, D-67157 Wachenheim (DE); FISCHER, Jakob, D-85414 Kirchdorf (DE); SIEGEL, Wolfgang, D-67117 Limburgerhof (DE)
(74) Vertreter: Kinzebach, Werner, Dr.
(86) Internationale Anmeldenummer: EP9901597
(87) Internationale Veröffentlichungsnummer: WO99046044

(56) Entgegenhaltungen:
- WO-A-99/13983
- US-A- 5 312 996
- US-A- 5 360 938
- US-A- 5 600 032
- PATENT ABSTRACTS OF JAPAN vol. 098, no. 001, 30. Januar 1998 & JP 09 255610 A (MITSUBISHI CHEM CORP), 30. September 1997

## Beschreibung

Die vorliegende Erfindung betrifft einen Katalysator, der einen Rh-Komplex mit einem zwei- oder mehrzähnigen Phosphonitliganden umfasst, worin der Phosphor und eines der Sauerstoffatome der Phosphonitgruppe Teil eines 5- bis 8-gliedrigen Heterocyclus sind, sowie ein Verfahren zur Hydroformylierung von Verbindungen, die wenigstens eine ethylenisch ungesättigte Doppelbindung enthalten, in Gegenwart eines solchen Katalysators.

Die Hydroformylierung oder Oxo-Synthese ist ein wichtiges großtechnisches Verfahren und dient der Herstellung von Aldehyden aus Olefinen, Kohlenmonoxid und Wasserstoff. Diese Aldehyde können gegebenenfalls im gleichen Arbeitsgang mit Wasserstoff zu den entsprechenden Oxo-Alkoholen hydriert werden. Die Reaktion selbst ist stark exotherm und läuft im Allgemeinen unter erhöhtem Druck und bei erhöhten Temperaturen in Gegenwart von Katalysatoren ab. Als Katalysatoren werden Co-, Rh- oder Ru-Verbindungen bzw. -komplexe eingesetzt, die zur Aktivitäts- und/oder Selektivitätsbeeinflussung mit amin- oder phosphinhaltigen Liganden modifiziert sein können. Zusätzliche Promotoren haben in der Praxis bisher keine Bedeutung erlangt. Bei der Hydroformylierungsreaktion kommt es aufgrund der möglichen CO-Anlagerung an jedes der beiden C-Atome einer Doppelbindung zur Bildung von isomeren Aldehydgemischen. Zusätzlich kann es beim Einsatz von internen Olefinen zu einer Doppelbindungsisomerisierung aus einer internen, in Richtung auf eine terminale Position kommen. In diesen isomeren Gemischen ist der n-Aldehyd im Allgemeinen vor dem iso-Aldehyd begünstigt, wobei jedoch aufgrund der wesentlich größeren technischen Bedeutung der n-Aldehyde eine Optimierung der Hydroformylierungskatalysatoren zur Erzielung einer größeren n-Selektivität angestrebt wird.

In Beller et al., Journal of Molecular Catalysis A, 104 (1995), Seiten 17-85, werden rhodiumhaltige, phosphinmodifizierte Katalysatoren zur Hydroformylierung von niedrig siedenden Olefinen beschrieben. Nachteilig an diesen Katalysatoren ist, dass sie nur unter Einsatz metallorganischer Reagenzien hergestellt werden können und die eingesetzten Liganden nur aufwendig und kostspielig hergestellt werden können. Zudem lassen sich mit diesen phosphinmodifizierten Katalysatoren interne, geradkettige und verzweigte Olefine sowie Olefine mit mehr als 7 Kohlenstoffatomen nur sehr langsam hydroformylieren.

Die WO 95/30680 beschreibt zweizähnige Phosphinliganden, bei denen die beiden Phosphingruppen an je einen Arylrest gebunden sind und diese beiden Arylreste ein zweifach verbrücktes, ortho-anelliertes Ringsystem bilden, wobei eine der beiden Brücken aus einem Sauerstoff- oder einem Schwefelatom besteht. Rhodiumkomplexe auf Basis dieser Liganden eignen sich als Hydroformylierungskatalysatoren, wobei bei der Hydroformylierung endständiger Olefine ein gutes n/iso-Verhältnis erzielt wird. Nachteilig an diesen Chelatphosphinen ist der hohe synthetische Aufwand zu ihrer Herstellung, so dass technische Verfahren, die auf solchen Chelatphosphinkatalysatoren beruhen, wirtschaftlich benachteiligt sind.

Die US-A-4,169,861 beschreibt ein Verfahren zur Herstellung endständiger Aldehyde durch Hydroformylierung von α-Olefinen in Gegenwart eines Rhodium-Hydroformylierungskatalysators auf Basis eines zweizähnigen und eines einzähnigen Liganden. Als zweizähniger Ligand wird dabei vorzugsweise 1,1'-Bis(diphenylphosphino)ferrocen eingesetzt. Bei dem einzähnigen Liganden handelt es sich vorzugsweise um Phosphine, wie Diphenylethylphosphin. Die US-A-4,201,714 und US-A-4,193,943 weisen einen vergleichbaren Offenbarungsgehalt auf. Die Herstellung der zweizähnigen Phosphinoferrocenliganden erfordert den Einsatz metallorganischer Reagenzien, die aufwendig in ihrer Herstellung sind, wodurch Hydroformylierungsverfahren unter Einsatz dieser Katalysatoren wirtschaftlich benachteiligt sind.

Die US-A-5,312,996 beschreibt ein Verfahren zur Herstellung von 1,6-Hexandial durch Hydroformylierung von Butadien in Gegenwart von Wasserstoff und Kohlenmonoxid. Als Hydroformylierungskatalysatoren werden Rhodiumkomplexe mit Polyphosphitliganden eingesetzt, worin der Phosphor und zwei der Sauerstoffatome der Phosphitgruppe Teil eines 7-gliedrigen Heterocyclus sind.

Die JP-A 97/255 610 beschreibt ein Verfahren zur Herstellung von Aldehyden durch Hydroformylierung in Gegenwart von Rhodiumkatalysatoren, die einen einzähnigen Phosphonitliganden aufweisen.

Keine der zuvor genannten Literaturstellen beschreibt Hydroformylierungskatalysatoren auf Basis von zwei- oder mehrzähnigen Phosphonitliganden, wobei die Phosphonitgruppe Teil eines 5- bis 8-gliedrigen Heterocyclus ist.

Der vorliegenden Erfindung liegt die Aufgabe zugrunde, neue Katalysatoren auf Basis von Rh-Komplexen zur Verfügung zu stellen. Diese sollen sich vorzugsweise zur Hydroformylierung eignen und eine gute katalytische Aktivität aufweisen.

Überraschenderweise wurden nun Katalysatoren auf Basis von Komplexen gefunden, welche mindestens einen zwei- oder mehrzähnigen Phosphonitliganden umfassen, wobei die Phosphonitgruppe Teil eines 5- bis 8-gliedrigen Heterocyclus ist.

Gegenstand der vorliegenden Erfindung ist somit ein Katalysator, umfassend einen Komplex, mit einem zwei- oder mehrzähnigen Phosphonitliganden der allgemeinen Formel I 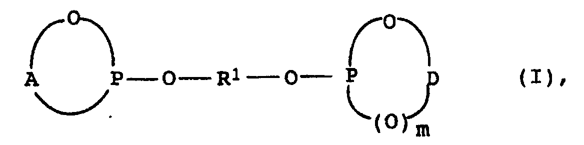 worin
- m: für 0 oder 1 steht,
- A: zusammen mit dem Teil der Phosphonitgruppe, an den es gebunden ist, für einen 5- bis 8-gliedrigen Heterocyclus steht, der gegebenenfalls zusätzlich ein-, zwei- oder dreifach mit Cycloalkyl, Aryl und/oder Hetaryl anelliert sein kann, wobei die anellierten Gruppen je einen, zwei oder drei Substituenten, ausgewählt unter Alkyl, Alkoxy, Halogen, Nitro, Cyano oder Carboxyl tragen können,
- R¹: für eine C₃- bis C₆-Alkylenbrücke steht, welche eine, zwei oder drei Doppelbindungen aufweisen und/oder ein-, zwei- oder dreifach mit Aryl und/oder Hetaryl anelliert sein kann, wobei die Aryl- oder Hetarylgruppen einen, zwei oder drei der fol- genden Substituenten: Alkyl, Cycloalkyl, Aryl, Alkoxy, Cycloalkyloxy, Aryloxy, Halogen, Trifluormethyl, Nitro, Cyano, Carboxyl oder NE¹E² tragen können, wobei E¹ und E² gleich oder verschieden sein können und für Alkyl, Cycloalkyl oder Aryl stehen,
- D: die zuvor für A angegebenen Bedeutungen besitzen kann,
oder Salze und Mischungen davon.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₈-Alkyl-, bevorzugter C₁-C₆-Alkyl- und besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1, 2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl.

Bei der Cycloalkylgruppe handelt es sich vorzugsweise um eine C₅-C₇-Cycloalkylgruppe, wie Cyclopentyl, Cyclohexyl oder Cyclo-heptyl.

Wenn die Cycloalkylgruppe substituiert ist, weist sie vorzugsweise 1, 2, 3, 4 oder 5, insbesondere 1, 2 oder 3 Substituenten, ausgewählt unter Alkyl, Alkoxy oder Halogen auf.

Aryl steht vorzugsweise für Phenyl, Tolyl, Xylyl, Mesityl, Naphthyl, Anthracenyl, Phenanthrenyl, Naphthacenyl und insbesondere für Phenyl oder Naphthyl.

Substituierte Arylreste weisen vorzugsweise 1, 2, 3, 4 oder 5, insbesondere 1, 2 oder 3 Substituenten, ausgewählt unter Alkyl, Alkoxy oder Halogen auf.

Hetaryl steht vorzugsweise für Pyridyl, Chinolinyl, Acridinyl, Pyridazinyl, Pyrimidinyl oder Pyrazinyl.

Substituierte Hetarylreste weisen vorzugsweise 1, 2 oder 3 Substituenten, ausgewählt unter Alkyl, Alkoxy oder Halogen auf.

Die obigen Ausführungen zu Alkyl-, Cycloalkyl- und Arylresten gelten entsprechend für Alkoxy-, Cycloalkyloxy- und Aryloxyreste.

Die Reste NE¹E² stehen vorzugsweise für N,N-Dimethyl, N,N-Diethyl, N,N-Dipropyl, N,N-Diisopropyl, N,N-Di-n-butyl, N,N-Di-t. -butyl, N,N-Dicyclohexyl oder N,N-Diphenyl.

Halogen steht für Fluor, Chlor, Brom und Iod, bevorzugt für Fluor, Chlor und Brom.

Eine bevorzugte Ausführungsform der Erfindung sind Katalysatoren, die mindestens einen Phosphonitliganden der Formel I umfassen, wobei A zusammen mit dem Teil der Phosphonitgruppe, an den es gebunden ist, für einen 5- oder 6-gliedrigen Heterocyclus steht, der gegebenenfalls ein- oder zweifach mit Aryl und/oder Hetaryl anelliert sein kann, wobei die anellierten Gruppen einen, zwei oder drei der zuvor angegebenen Substituenten tragen können.

Der Rest A steht dann z. B. für einen 2,2'-Biphenylen-, 2,2'-Binaphthylen- oder 2,3-Xylylen-Rest, der 1, 2 oder 3 Substituenten, ausgewählt unter Alkyl, Alkoxy oder Halogen, tragen kann. Alkyl steht dabei vorzugsweise für C₁-C₄-Alkyl und insbesondere für t.-Butyl. Alkoxy steht dabei vorzugsweise für C₁-C₄-Alkoxy und insbesondere für Methoxy. Halogen steht insbesondere für Fluor, Chlor oder Brom.

Bevorzugt steht R¹ für einen Rest der Formeln II.1, II.2, II.3 oder II.4: 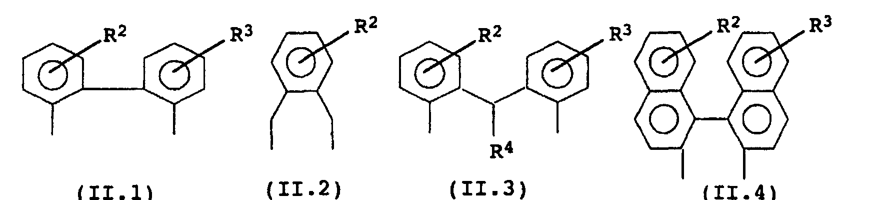 worin
- R2 und R³: unabhängig voneinander für Wasserstoff, Alkyl, Alkoxy, Halogen, Trifluormethyl, Nitro oder Cyano stehen, und
- R⁴: für Wasserstoff, Alkyl, vorzugsweise Methyl, oder Aryl, vorzugsweise Phenyl, steht, welches gegebenenfalls mit Alkyl, Alkoxy, Halogen, Trifluormethyl, Nitro oder Cyano substituiert sein kann.

Die obigen Ausführungen zu bevorzugten Resten A gelten entsprechend für Reste D.

Nach einer geeigneten Ausführungsform sind die Phosphonitliganden der Formel I ausgewählt unter Liganden der Formel Ia bis Ig

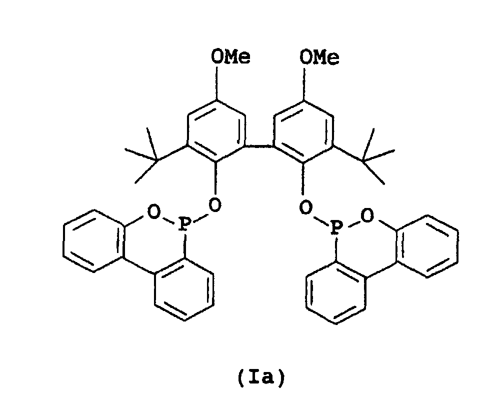

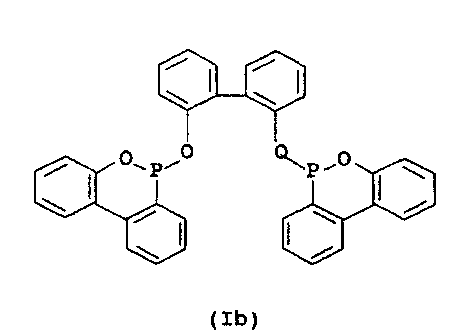

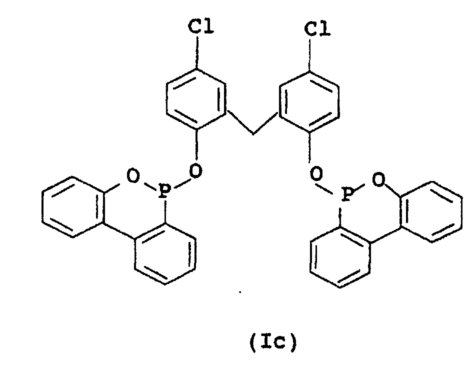

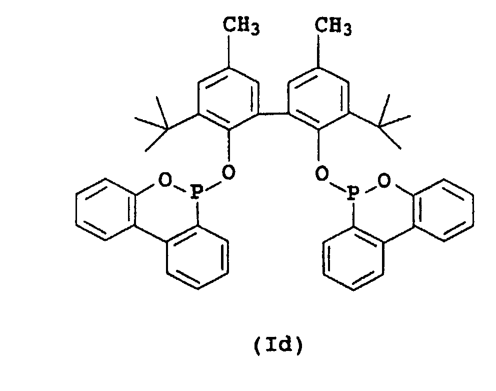

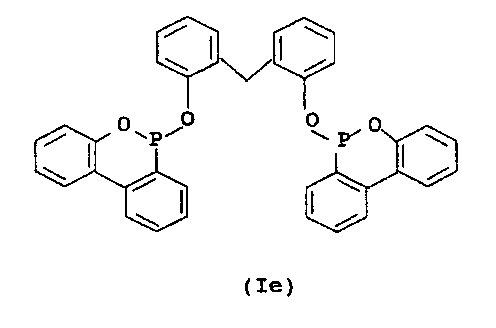

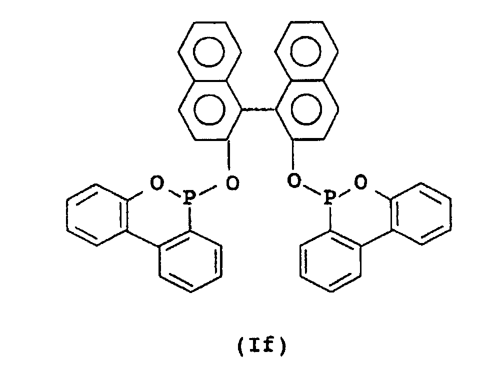

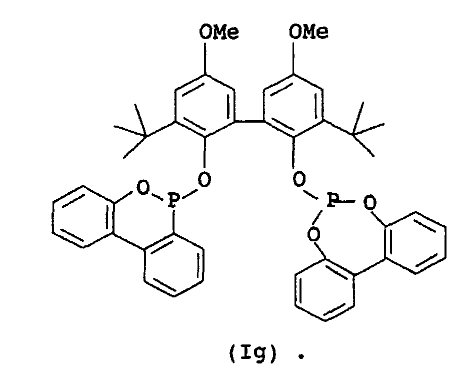

Die erfindungsgemäßen Katalysatoren können einen oder mehrere der Phosphonitliganden der Formel I aufweisen. Zusätzlich zu den zuvor beschriebenen Liganden der allgemeinen Formel I können sie noch wenigstens einen weiteren Liganden, der ausgewählt ist unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein-, zwei- und mehrzähnigen Phosphin-, Phosphinit-, Phosphonit- und Phosphitliganden aufweisen. Diese weiteren Liganden können ebenfalls ein-, zwei- oder mehrzähnig sein und am Metallatom des Katalysatorkomplexes koordinieren. Geeignete weitere phosphorhaltige Liganden sind z. B. übliche Phosphin-, Phosphinit-, und Phosphitliganden.

Zur Herstellung der erfindungsgemäß eingesetzten Phosphonitliganden der Formel I kann man z. B. eine Hydroxylgruppen-haltige Verbindung der Formel III mit einem Phosphortrihalogenid, bevorzugt PCl₃, zu einer Verbindung der Formel IV und diese dann mit einer Hydroxylgruppen-haltigen Verbindung der Formel HOR¹OH und einer Verbindung der Formel V gemäß folgendem Schema 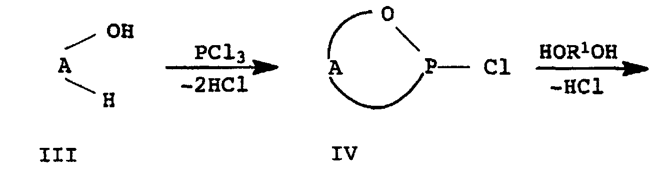 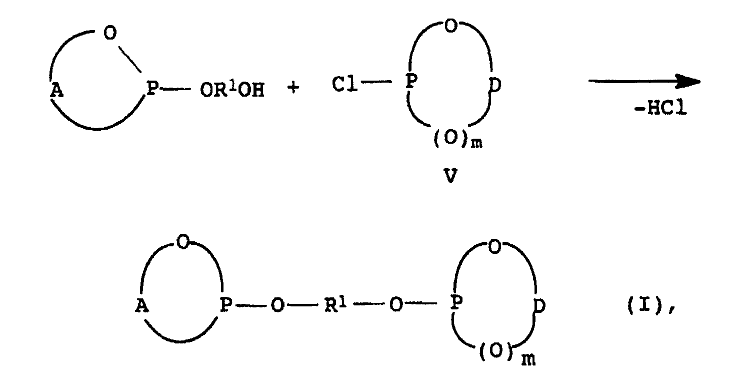 umsetzen,
wobei m, A, D und R¹ die zuvor angegebenen Bedeutungen besitzen. Gewünschtenfalls können auch 2 Mol einer Verbindung der Formel IV mit einem Mol einer Verbindung HOR¹OH zu einem zweizähnigen Phosphonitliganden mit zwei gleichen Phosphonitresten umgesetzt werden. Ein Verfahren zur Herstellung dieser Liganden wird in Phosphorus and Sulfur, 1987, Bd. 31, S. 71 ff. für den Aufbau von 6H-Dibenz[c,e][1,2]oxaphosphorin-Ringsystemen beschrieben.

Geeignete Alkohole der Formel HOR¹OH sind z. B. Biphenyl-2,2'-diol und Binaphthyl-2,2'-diol. Weitere geeignete Diole werden in der US-A-5,312,996, Sp. 19 genannt, auf die hier Bezug genommen wird. Zur Herstellung von zweizähnigen Liganden der Formel I, welche eine Phosphonit- und eine Phosphitgruppe tragen, kann man eine Verbindung der Formel IV mit einer Verbindung der Formel HOR¹OH zu einem Monokondensationsprodukt umsetzen und dieses dann mit einer Verbindunq der Formel V 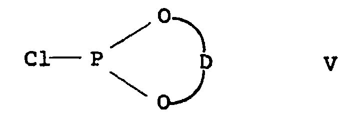 worin D die zuvor für A angegebenen Bedeutungen besitzen kann, zu einem gemischten Liganden der Formel I umsetzen.

Die Verbindungen der Formel IV können gewünschtenfalls isoliert und einer Reinigung, z. B. durch Destillation, unterworfen werden. Die Umsetzung der Verbindung der Formel III zu einer Verbindung der Formel IV verläuft im Allgemeinen bei einer erhöhten Temperatur in einem Bereich von etwa 40 bis etwa 200 °C, wobei die Umsetzung auch unter sukzessiver Temperaturerhöhung geführt werden kann. Zusätzlich kann zu Beginn der Reaktion oder nach einer gewissen Reaktionsdauer eine Lewis-Säure, wie z. B. Zinkchlorid oder Aluminiumchlorid, als Katalysator zugesetzt werden. Die weitere Umsetzung der Verbindungen der Formel IV zu den erfindungsgemäß eingesetzten Phosphonitliganden der Formel I erfolgt im Allgemeinen in Gegenwart einer Base, z. B. einem aliphatischen Amin, wie Diethylamin, Dipropylamin, Dibutylamin, Trimethylamin, Tripropylamin und vorzugsweise Triethylamin oder Pyridin.

Vorteilhafterweise gelingt die Herstellung der erfindungsgemäß eingesetzten Phosphonitliganden der Formel I ohne Verwendung von Magnesium- oder Lithium-organischen Verbindungen. Die einfache Reaktionssequenz erlaubt eine breite Variationsmöglichkeit der Liganden. Die Darstellung gelingt somit effizient und ökonomisch aus leicht zugängigen Edukten.

Im Allgemeinen werden unter Hydroformylierungsbedingungen aus den jeweils eingesetzten Katalysatoren oder Katalysatorvorstufen katalytisch aktive Spezies der allgemeinen Formel HₓRh_{y}(CO)_{z}L_{q} gebildet, worin L für einen erfindungsgemäßen Phosphonitliganden und q, x, y, z für ganze Zahlen, abhängig von der Wertigkeit des Rhodium sowie der Bindigkeit des Liganden L, stehen. Vorzugsweise stehen z und q unabhängig voneinander mindestens für einen Wert von 1, wie z. B. 1, 2 oder 3. Die Summe aus z und q steht bevorzugt für einen Wert von 2 bis 5. Dabei können die Komplexe gewünschtenfalls zusätzlich noch mindestens einen der zuvor beschriebenen weiteren Liganden aufweisen.

Nach einer bevorzugten Ausführungsform werden die Hydroformylierungskatalysatoren in situ, in dem für die Hydroformylierungsreaktion eingesetzten Reaktor, hergestellt. Gewünschtenfalls können die erfindungsgemäßen Katalysatoren jedoch auch separat hergestellt und nach üblichen Verfahren isoliert werden. Zur in situ-Herstellung der erfindungsgemäßen Katalysatoren setzt man wenigstens einen Phosphonitliganden der allgemeinen Formel I, eine Rh-Verbindung oder einen Rh-Komplex, gegebenenfalls wenigstens einen weiteren zusätzlichen Liganden und gegebenenfalls ein Aktivierungsmittel in einem inerten Lösungsmittel unter den Hydroformylierungsbedingungen um.

Geeignete Rhodiumveroindungen oder -komplexe sind z. B. Rhodium(II)- und Rhodium(III)-salze, wie Rhodium(III)-chlorid, Rhodium( III) -nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(III)-oxid, Salze der Rhodium(III)-säure, Trisammoniumhexachlororhodat(III) etc. Weiterhin eignen sich Rhodiumkomplexe, wie Rhodiumbiscarbonylacetylacetonat, Acetylacetonatobisethylenrhodium(I) etc. Vorzugsweise werden Rhodiumbiscarbonylacetylacetonat oder Rhodiumacetat eingesetzt.

Die genannten Verbindungen des Rhodiums sind im Prinzip bekannt und in der Literatur hinreichend beschrieben oder sie können vom Fachmann analog zu den bereits bekannten Verbindungen hergestellt werden.

Geeignete Aktivierungsmittel sind z. B. Brönsted-Säuren, LewisSäuren, wie z. B. BF₃, AlCl₃, ZnCl₂ und Lewis-Basen.

Als Lösungsmittel werden vorzugsweise die Aldehyde eingesetzt, die bei der Hydroformylierung der jeweiligen Olefine entstehen, sowie deren höher siedende Folgereaktionsprodukte, z. B. die Produkte der Aldolkondensation. Bei ausreichend hydrophilisierten Liganden können auch Wasser, Alkohole, wie Methanol, Ethanol, n-Propanol, Isopropanol, n-Butanol, Isobutanol, Ketone, wie Aceton und Methylethylketon etc. eingesetzt werden.

Das Molmengenverhältnis von Phosphonitligand der allgemeinen Formel I zu Metall der VIII. Nebengruppe liegt im Allgemeinen in einem Bereich von etwa 1:1 bis 1 000:1.

Ein weiterer Gegenstand der Erfindung ist ein Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart wenigstens eines der erfindungsgemäßen Hydroformylierungskatalysatoren.

Als Substrate für das erfindungsgemäße Hydroformylierungsverfahren kommen prinzipiell alle Verbindungen in Betracht, welche eine oder mehrere ethylenisch ungesättigte Doppelbindungen enthalten. Dazu zählen z. B. Olefine, wie α-Olefine, interne geradkettige und interne verzweigte Olefine. Geeignete α-Olefine sind z. B. Ethylen, Propen, 1-Buten, 1-Penten, 1-Hexen, 1-Hepten, 1-Octen, 1-Nonen, 1-Decen, 1-Undecen, 1-Dodecen etc.

Geeignete geradkettige interne Olefine sind vorzugsweise C₄- bis C₂₀-Olefine, wie 2-Buten, 2-Penten, 2-Hexen, 3-Hexen, 2-Hepten, 3-Hepten, 2-Octen, 3-Octen, 4-Octen etc.

Geeignete verzweigte, interne Olefine sind vorzugsweise C₄- bis C₂₀-Olefine, wie 2-Methyl-2-Buten, 2-Methyl-2-Penten, 3-Methyl-2-Penten, verzweigte, interne Hepten-Gemische, verzweigte, interne Octen-Gemische, verzweigte, interne Nonen-Gemische, verzweigte, interne Decen-Gemische, verzweigte, interne Undecen-Gemische, verzweigte, interne Dodecen-Gemische etc.

Geeignete zu hydroformylierende Olefine sind weiterhin C₅- bis C₈-Cycloalkene, wie Cyclopenten, Cyclohexen, Cyclohepten, Cycloocten und deren Derivate, wie z. B. deren C₁- bis C₂₀-Alkylderivate mit 1 bis 5 Alkylsubstituenten. Geeignete zu hydroformylierende Olefine sind weiterhin Vinylaromaten, wie Styrol, α-Methylstyrol, 4-Isobutylstyrol etc. Geeignete zu hydroformylierende Olefine sind weiterhin α,β-ethylenisch ungesättigte Mono- und/oder Dicarbonsäuren, deren Ester, Halbester und Amide, wie Acrylsäure, Methacrylsäure, Maleinsäure, Fumarsäure, Crotonsäure, Itaconsäure, 3-Pentensäuremethylester, 4-Pentensäuremethylester, Ölsäuremethylester, Acrylsäuremethylester, Methacrylsäuremethylester, ungesättigte Nitrile, wie 3-Pentennitril, 4-Pentennitril, Acrylnitril, Vinylether, wie Vinylmethylether, Vinylethylether, Vinylpropylether etc., C₁- bis C₂₀-Alkenole, -Alkendiole und -Alkadienole, wie 2,7-Octadienol-1. Geeignete Substrate sind weiterhin Di- oder Polyene mit isolierten oder konjugierten Doppelbindungen. Dazu zählen z. B. 1,3-Butadien, 1,4-Pentadien, 1,5-Hexadien, 1,6-Heptadien, 1,7-Octadien, Vinylcyclohexen, Dicyclopentadien, 1,5,9-Cyclooctatrien sowie Butadienhomo- und -copolymere.

Die Hydroformylierungsreaktion kann kontinuierlich, semikontinuierlich oder diskontinuierlich erfolgen.

Geeignete Reaktoren für die kontinuierliche Umsetzung sind dem Fachmann bekannt und werden z. B. in Ullmanns Enzyklopädie der technischen Chemie, Bd. 1, 3. Aufl., 1951, S. 743 ff. beschrieben.

Geeignete druckfeste Reaktoren sind dem Fachmann ebenfalls bekannt und werden z. B. in Ullmanns Enzyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die Zusammensetzung des im erfindungsgemäßen Verfahren eingesetzten Synthesegases aus Kohlenmonoxid und Wasserstoff kann in weiten Bereichen variieren. Das molare Verhältnis von Kohlenmonoxid und Wasserstoff beträgt in der Regel etwa 5:95 bis 70:30, bevorzugt etwa 40:60 bis 60:40. Insbesondere bevorzugt wird ein molares Verhältnis von Kohlenmonoxid und Wasserstoff im Bereich von etwa 1:1 eingesetzt.

Die Temperatur bei der Hydroformylierungsreaktion liegt im Allgemeinen in einem Bereich von etwa 20 bis 180 °C, bevorzugt etwa 50 bis 150 °C. Die Reaktion wird in der Regel bei dem Partialdruck des Reaktionsgases bei der gewählten Reaktionstemperatur durchgeführt. Im Allgemeinen liegt der Druck in einem Bereich von etwa 1 bis 700 bar, bevorzugt 1 bis 600 bar, insbesondere 1 bis 300 bar. Der Reaktionsdruck kann in Abhängigkeit von der Aktivität des eingesetzten erfindungsgemäßen Hydroformylierungskatalysators variiert werden. Im Allgemeinen erlauben die erfindungsgemäßen Katalysatoren auf Basis von Phosphonitliganden eine Umsetzung in einem Bereich niedriger Drücke, wie etwa im Bereich von 1 bis 100 bar.

Die erfindungsgemäßen Hydroformylierungskatalysatoren lassen sich nach üblichen, dem Fachmann bekannten Verfahren vom Austrag der Hydroformylierungsreaktion abtrennen und können im Allgemeinen erneut für die Hydroformylierung eingesetzt werden.

Vorteilhafterweise zeigen die erfindungsgemäßen Katalysatoren eine hohe Aktivität, so dass in der Regel die entsprechenden Aldehyde in guten Ausbeuten erhalten werden. Bei der Hydroformylierung von α-Olefinen sowie von innenständigen, linearen Olefinen zeigen sie zudem eine sehr geringe Selektivität zum Hydrierprodukt des eingesetzten Olefins.

Die zuvor beschriebenen, erfindungsgemäßen Katalysatoren, die chirale Phosphonitliganden der Formel I umfassen, eignen sich zur enantioselektiven Hydroformylierung.

Die Erfindung wird anhand der folgenden, nicht einschränkenden Beispiele näher erläutert.

### Beispiele

### A) Herstellung der Liganden Ia bis Ig

### Beispiel 1:

### Herstellung von Ligand Ia

206 g (1,5 mol) Phosphortrichlorid und 204 g (1,2 mol) Biphenyl-2-ol werden unter Rühren in einer Argonatmosphäre langsam auf 50 °C und innerhalb von 8 Stunden weiter auf 140 °C erhitzt. Bei starker Chlorwasserstoffentwicklung färbt sich die Lösung gelb. Nach Abkühlen auf 120 °C fügt man eine katalytische Menge an Zinkchlorid (1,2 g; 17 mmol) zu und erhitzt 24 Stunden bei 140 °C. Bei anschließender Destillation geht das Reaktionsprodukt 6-Chlor-(6H)-dibenz [c,e] [1,2]-oxaphosphorin bei einem Siedepunkt von 132 °C (0,2 mbar) über. Ausbeute: 194,8 g (69 %) weiße Kristalle; ³¹P-NMR-Spektrum: 6 (ppm) 134,5.

40 g (0,177 mol) 6-Chlor-(6H)-dibenz[c,e][1,2]-oxaphosphorin werden unter Argon zusammen mit 31,7 g (0,088 mol) 4,4'-Methoxy-6,6'-t.-butyl-2,2'-biphenol in 400 ml Toluol vorgelegt. Bei Raumtemperatur werden 20,24 g (0,2 mol) Triethylamin (über KOH getrocknet) zugetropft. Anschließend rührt man 120 Minuten bei 90 °C nach. Das entstandene Triethylammoniumhydrochlorid wird abfiltriert und der Filterrückstand zur Vervollständigung der Ausbeute mit Tetrahydrofuran nachgewaschen. Von den vereinigten organischen Phasen werden die flüchtigen Bestandteile in Hochvakuum entfernt. Als Produkt erhält man den Liganden Ia in 100 % Rohausbeute. Der weißgelbe Feststoff wird zunächst mit n-Hexan und dann mit Diethylether gewaschen. 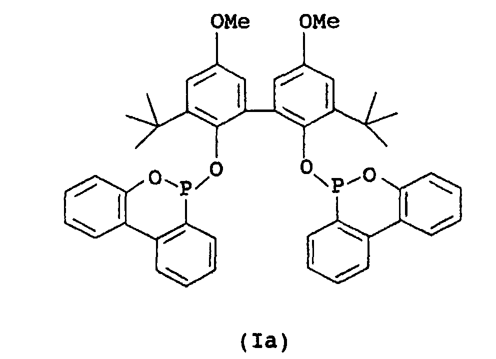 ³¹P-NMR-Spektrum: δ (ppm) 128,14

### Beispiel 2:

### Herstellung von Ligand Ic

Analog der in Beispiel 1 angegebenen Synthesevorschrift erfolgt die Herstellung des Liganden Ic. Das erhaltene Rohprodukt weist eine braune Farbe auf und ist leicht klebrig. Es wird zur Reinigung 12 Stunden in n-Hexan kräftig gerührt. Nach Abtrennen der überstehenden Hexanlösung erhält man den Liqanden Ic als weißes Pulver. 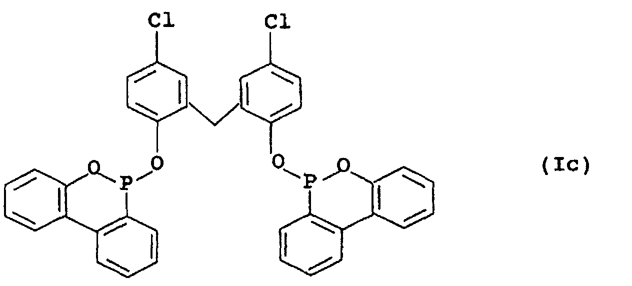 ³¹P-NMR-Spektrum: δ (ppm) 128,41
¹H-NMR-Spektrum: entspricht dem Strukturvorschlag
Reinheit Rohprodukt: > 89 %

### Beispiel 3:

### Herstellung von Ligand If

In 200 ml Toluol werden bei Raumtemperatur in einer Argonatmosphäre 7,95 g (33,8 mmol) 6-Chlor-(6H)-dibenz[c,e][1,2]-oxaphosphorin und 4,84 g (16,9 mmol) 2,2'-Dihydroxy-1,1'-dinaphthyl vorgelegt. Bei Raumtemperatur werden innerhalb von 10 Minuten 4,28 g (42,2 mmol) Triethylamin zugetropft. Anschließend rührt man eine Stunde bei 90 °C nach. Das entstandene Triethylammoniumhydrochlorid wird abfiltriert, die flüchtigen Bestandteile werden im Hochvakuum entfernt. Zurück bleiben 11,5 g eines schwach gelblich gefärbten Feststoffes (99,6 % Rohausbeute).

Um Spuren von Verunreinigungen zu entfernen, wird der Feststoff mehrfach mit geringen Mengen an kaltem Methyl-tert.-butylether gewaschen. Der zurückbleibende Feststoff wird in entgastem Methylenchlorid aufgenommen. Die organische Lösung wird mehrfach mit entgastem Wasser extrahiert, über Natriumsulfat getrocknet und eingeengt. Es verbleibt ein weißer Feststoff. 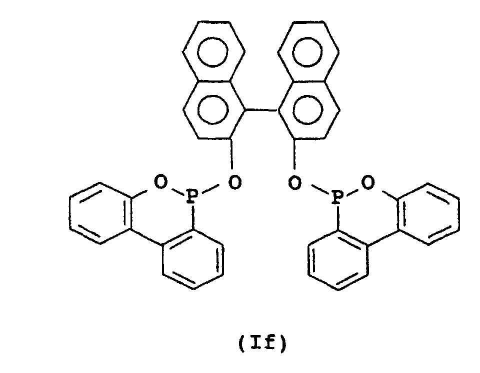 ³¹P-NMR-Spektrum: δ (ppm) 131,20; 130,01; 128,75; 127,15 im Verhältnis 1:1:1:1 (Stereoisomere)
Reinheit Rohprodukt: 97,6 %
¹H-NMR: entspricht dem Strukturvorschlag

### Beispiel 4:

### Herstellung von Ligand Ib

Analog der in Beispiel 3 angegebenen Vorschrift wurde Ligand Ib hergestellt und als weißer Feststoff erhalten.
³¹P-NMR-Spektrum: δ (ppm) 128,20
¹H-NMR: entspricht dem Strukturvorschlag
Reinheit Rohprodukt: > 99 %

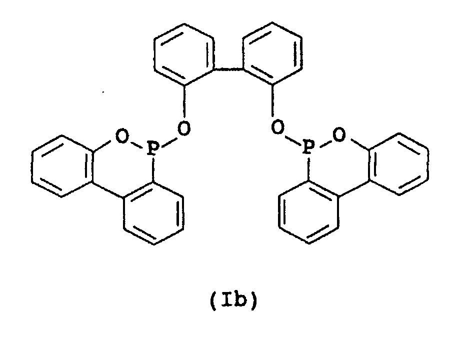

### Beispiel 5:

### Herstellung von Ligand Ie

Analog der in Beispiel 3 angegebenen Vorschrift wurde Ligand Ie hergestellt und als weißer Feststoff erhalten.
³¹P-NMR-Spektrum: δ (ppm) 127,2
¹H-NMR: entspricht dem Strukturvorschlag
Reinheit Rohprodukt: > 97 %

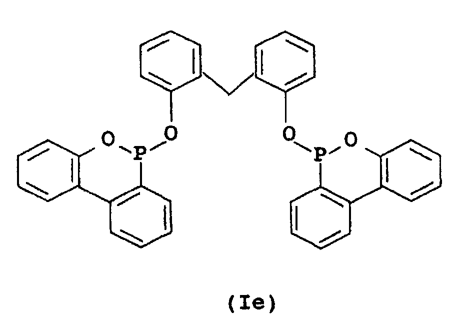

### B) Hydroformylierungen

### Beispiel 6:

### Hydroformylierung von 3-Pentennitril

In einem 10 ml-Stahlautoklaven wurden unter Argonschutzgas 0,75 mg Rhodiumbiscarbonylacetylacatonat, 12,3 mg Ligand If, 1,5 g 3-Pentennitril und 1,5 g Xylol bei 100 °C mit einem Synthesegasgemisch CO/H₂ (1:1) bei 80 bar umgesetzt. Nach einer Reaktionszeit von 4 Stunden wurde der Autoklav entspannt und entleert. Das Gemisch wurde mittels GC mit internem Standard analysiert. Der Umsatz betrug 58 %. Die Ausbeuten betrugen 57 % Formylvaleronitril-Isomere (12 % n-Anteil), 1,2 % Pentannitril.

## Patentansprüche

1. Katalysator, umfassend einen Rhodium-Komplex mit einem zwei- oder mehrzähnigen Phosphonitliganden der allgemeinen Formel I 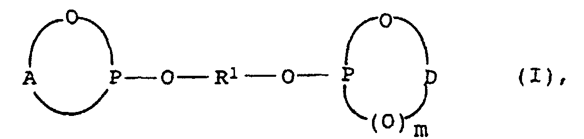 worin
m für 0 oder 1 steht,
A zusammen mit dem Teil der Phosphonitgruppe, an den es gebunden ist, für einen 5- bis 8-gliedrigen Heterocyclus steht, der gegebenenfalls zusätzlich ein-, zwei- oder dreifach mit Cycloalkyl, Aryl und/oder Hetaryl anelliert sein kann, wobei die anellierten Gruppen je einen, zwei oder drei Substituenten, ausgewählt unter Alkyl, Alkoxy, Halogen, Nitro, Cyano oder Carboxyl tragen können,
R¹ für eine C₃- bis C₆-Alkylenbrücke steht, welche eine, zwei oder drei Doppelbindungen aufweisen und/oder ein-, zwei- oder dreifach mit Aryl und/oder Hetaryl anelliert sein kann, wobei die Aryl- oder Hetarylgruppen einen, zwei oder drei der folgenden Substituenten: Alkyl, Cycloalkyl, Aryl, Alkoxy, Cycloalkyloxy, Aryloxy, Halogen, Trifluormethyl, Nitro, Cyano, Carboxyl oder NE¹E² tragen können, wobei E¹ und E² gleich oder verschieden sein können und für Alkyl, Cycloalkyl oder Aryl stehen,
D die zuvor für A angegebenen Bedeutungen besitzen kann,
oder Salze und Mischungen davon.

2. Katalysator nach Anspruch 1, wobei R¹ für einen Rest der Formel II.1, II.2, II.3 oder II.4 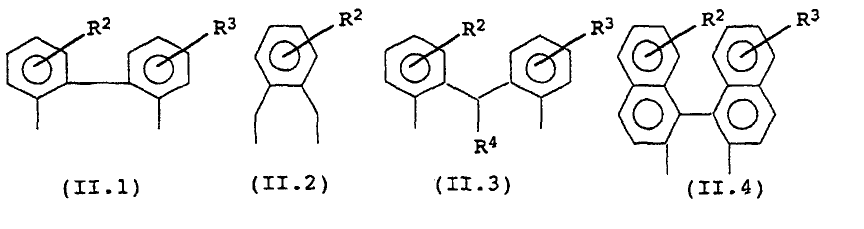 steht, worin
R² und R³ unabhängig voneinander für Wasserstoff, Alkyl, Alkoxy, Halogen, Trifluormethyl, Nitro oder Cyano stehen,
R⁴ für Wasserstoff, Alkyl, vorzugsweise Methyl, oder Aryl, vorzugsweise Phenyl, steht, welches gegebenenfalls durch Alkyl, Alkoxy, Halogen, Trifluormethyl, Nitro oder Cyano substituiert sein kann.

3. Katalysator nach einem der vorhergehenden Ansprüche, wobei der Phosphonitligand der Formel I ausgewählt ist unter Liganden der Formeln Ia bis Ig

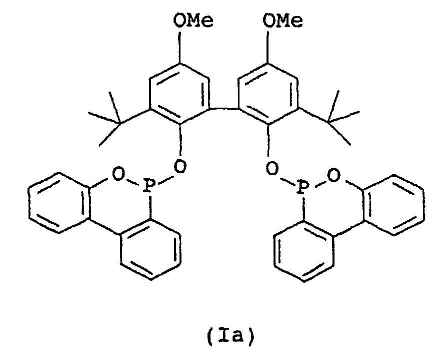

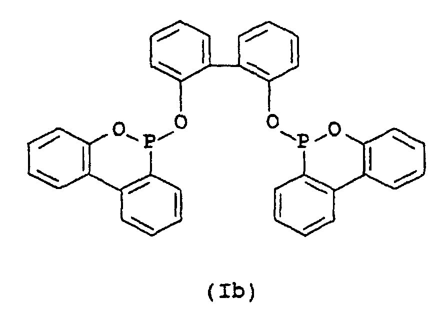

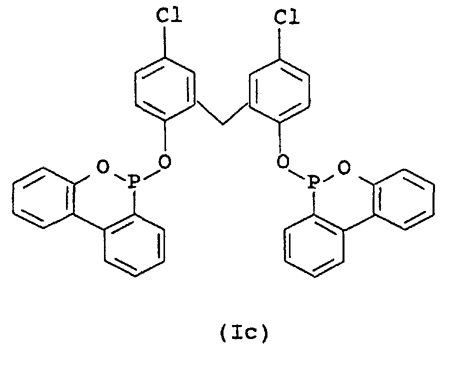

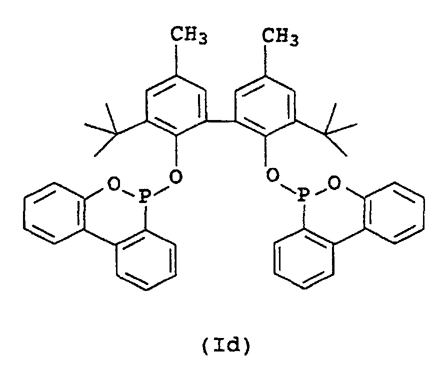

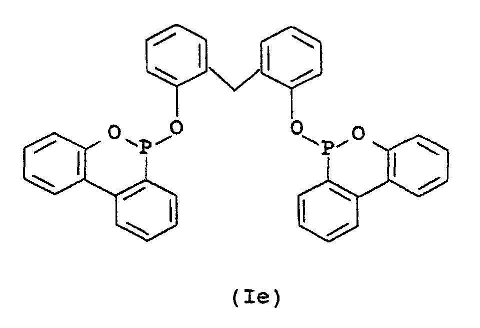

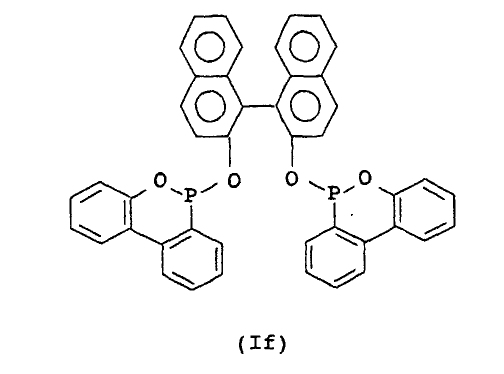

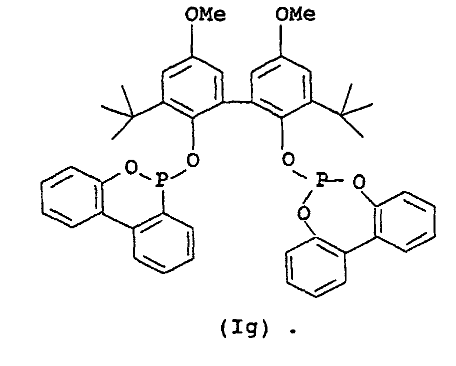

4. Katalysator nach einem der vorhergehenden Ansprüche, der zusätzlich wenigstens einen weiteren Liganden, ausgewählt unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein-, zwei- und mehrzähnigen Phosphin-, Phosphinit- und Phosphitliganden aufweist.

5. Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Hydroformylierungskatalysators, **dadurch gekennzeichnet, dass** man als Hydroformylierungskatalysator einen Katalysator nach einem der Ansprüche 1 bis 4 einsetzt.

6. Verfahren nach Anspruch 5, **dadurch gekennzeichnet, dass** der Hydroformylierungskatalysator in situ hergestellt wird, wobei man mindestens einen Phosphonitliganden der allgemeinen Formel I, wie in den Ansprüchen 1 bis 3 definiert, eine Verbindung oder einen Komplex eines Metalls der VIII. Nebengruppe und gegebenenfalls ein Aktivierungsmittel in einem inerten Lösungsmittel unter den Hydroformylierungsbedingungen zur Reaktion bringt.

7. Verwendung von Katalysatoren, umfassend einen Phosphonitliganden der Formel I, gemäß einem der Ansprüche 1 bis 3 zur Hydroformylierung.

## Claims

1. A catalyst comprising a rhodium complex with a bi- or more highly dentate phosphonite ligand of the general formula I 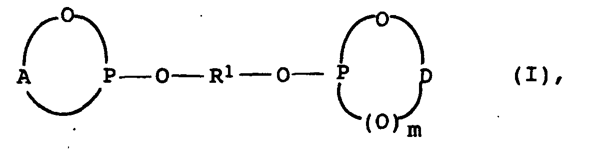 where
m is 0 or 1,
A combines with that part of the phosphonite group to which it is attached to form a 5- to 8-membered heterocycle which may. optionally be additionally singly, doubly or triply fused with cycloalkyl, aryl and/or hetaryl, in which case the fused-on groups may each bear one, two or three substituents selected from the group consisting of alkyl, alkoxy, halogen, nitro, cyano and carboxyl,
R¹ is a C₃- to C₆-alkylene bridge which may have one, two or three double bonds and/or may be singly, doubly or triply fused with aryl and/or hetaryl, in which case the aryl or hetaryl groups may bear one, two or three substituents selected from the group consisting of alkyl, cycloalkyl, aryl, alkoxy, cycloalkyloxy, aryloxy, halogen, trifluoromethyl, nitro, cyano, carboxyl and NE¹E², where E¹ and E² are identical or different and each is alkyl, cycloalkyl or aryl,
D has the meanings specified above for A,
or salts and mixtures thereof.

2. A catalyst as claimed in claim 1, wherein R¹ is a radical of the formula II.1, II.2, II.3 or II.4 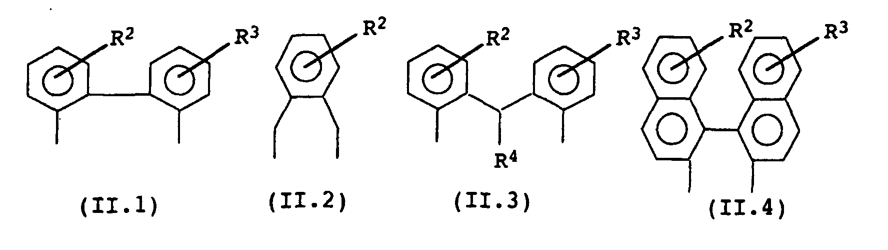 where
R² and R³ are independently hydrogen, alkyl, alkoxy, halogen, trifluoromethyl, nitro or cyano, and
R⁴ is hydrogen, alkyl, preferably methyl, or aryl, preferably phenyl, which may optionally be substituted by alkyl, alkoxy, halogen, trifluoromethyl, nitro or cyano.

3. A catalyst as claimed in either of the preceding claims, wherein the phosphonite ligand of the formula I is selected from the group consisting of ligands of the formulae Ia to Ig

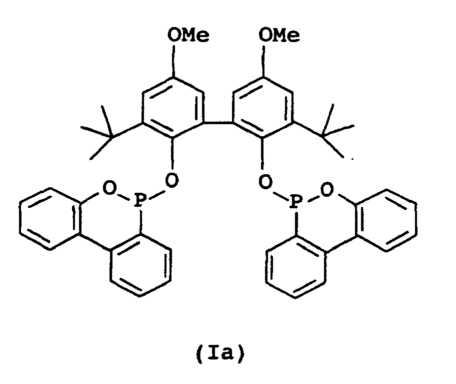

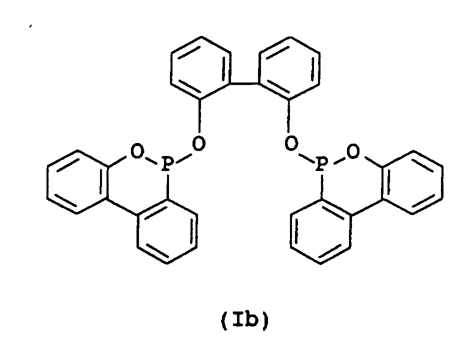

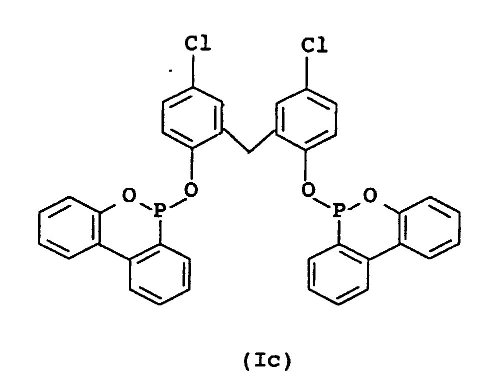

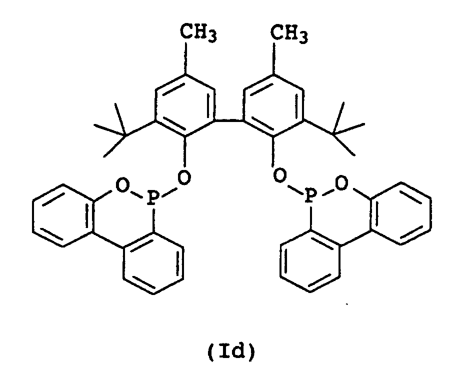

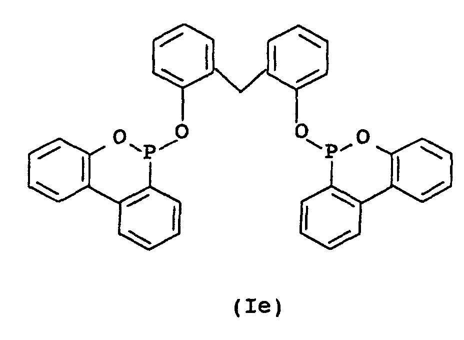

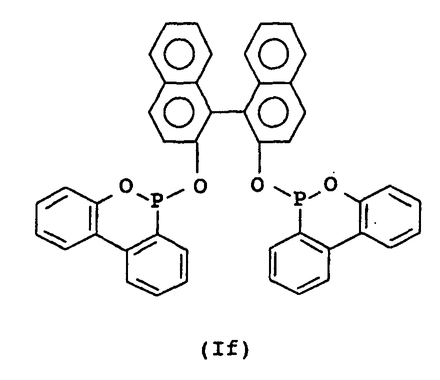

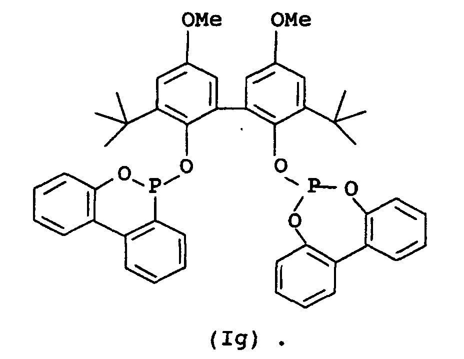

4. A catalyst as claimed in any of the preceding claims, further comprising at least one further ligand selected from the group consisting of halides, amines, carboxylates, acetylacetonate, arylsulfonates, alkylsulfonates, hydride, CO, olefins, dienes, cycloolefins, nitriles, N-containing heterocycles, aromatics, heteroaromatics, ethers, PF₃ and mono-, bi- and more highly dentate phosphine, phosphinite and phosphite ligands.

5. A process for hydroformylating compounds containing at least one ethylenically unsaturated double bonds by reaction with carbon monoxide and water in the presence of a hydrogen formylation catalyst, which comprises using a hydroformylation catalyst comprising a catalyst as claimed in any of claims 1 to 4.

6. A process as claimed in claim 5, wherein the hydroformylation catalyst is prepared in situ by reacting at least one phosphonite ligand of the general formula I as defined in claims 1 to 3, a compound or a complex of a metal of the VIIIth transition group and optionally an activating agent in an inert solvent under the hydroformylation conditions.

7. The use of catalysts comprising a phosphonite ligand of the formula I as claimed in any of claims 1 to 3 for hydroformylation.

## Revendications

1. Catalyseur comprenant un complexe du rhodium, avec un ligand phosphonite bi- ou multidenté de formule générale I 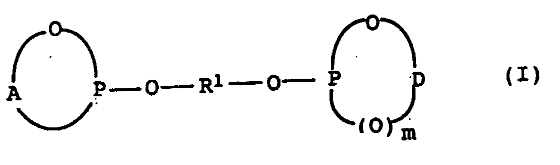 dans laquelle
m vaut 0 ou 1,
A, avec la partie du groupe phosphonite à laquelle il est lié, représente un radical hétérocyclique à 5 ou 8 chaînons, qui éventuellement peut en outre être condensé une, deux ou trois fois à des radicaux cycloalkyle, aryle et/ou hétéroaryle, chacun des groupes condensés pouvant porter un, deux ou trois substituants choisis parmi les groupes alkyle, alcoxy, halogéno, nitro, cyano ou carboxyle,
R¹ est un pont alkylène en C₃ à C₆, qui peut comporter un, deux ou trois doubles liaisons et/ou peut être condensé une fois, deux fois ou trois fois à des radicaux aryle et/ou hétéroaryle, les groupes aryle ou hétéroaryle pouvant porter un, deux ou trois des substituants suivants : alkyle, cycloalkyle, aryle, alcoxy, cycloalkyloxy, aryloxy, halogéno, trifluorométhyle, nitro, cyano, carboxyle ou NE¹E², où E¹ et E² peuvent être identiques ou différents et représentent chacun un groupe alkyle, cycloalkyle ou aryle,
D peut avoir les significations données ci-dessus pour A,
ou leurs sels et mélanges.

2. Catalyseur selon la revendication 1, dans lequel R¹ représente un radical de formule II. 1, II.2, II.3 ou II.4 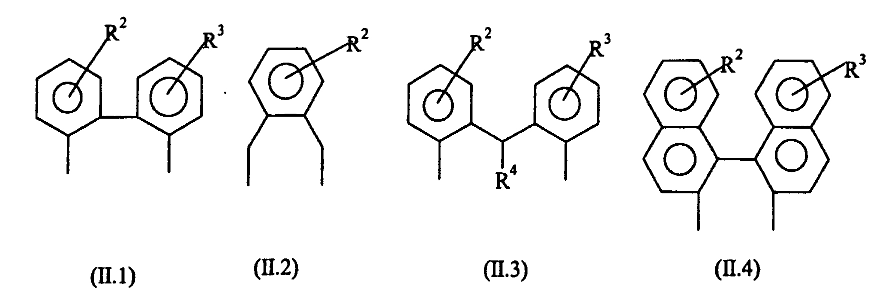 dans lesquelles
R² et R³ représentent chacun indépendamment de l'autre un atome d'hydrogène ou un groupe alkyle, alcoxy, halogéno, trifluorométhyle, nitro ou cyano,
R⁴ est un atome d'hydrogène ou un groupe alkyle, de préférence méthyle, ou aryle, de préférence phényle, qui peut éventuellement être substitué par des substituants alkyle, alcoxy, halogéno, trifluorométhyle, nitro ou cyano.

3. Catalyseur selon l'une des revendications précédentes, dans lequel le ligand phosphonite de formule I est choisi parmi les ligands ayant les formules Ia à Ig

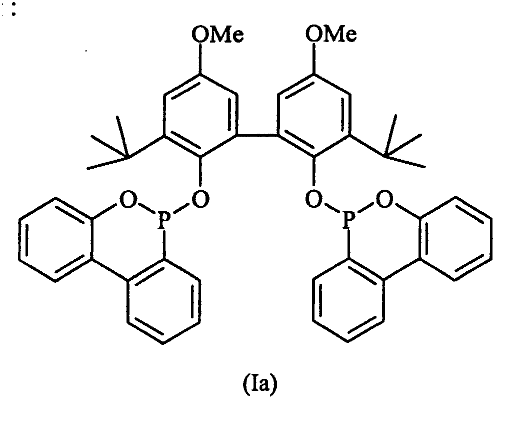

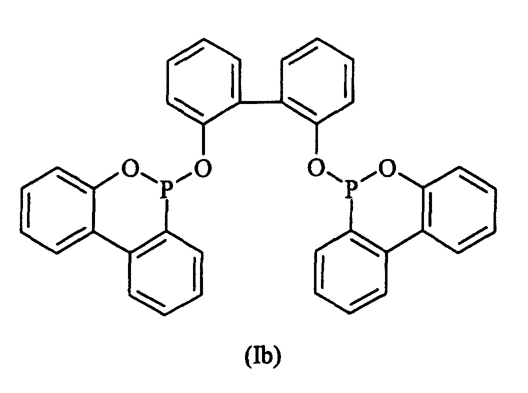

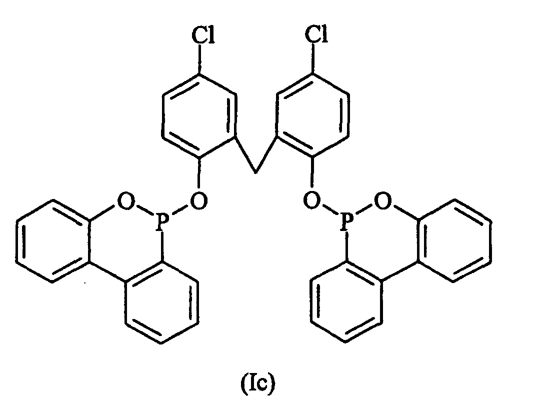

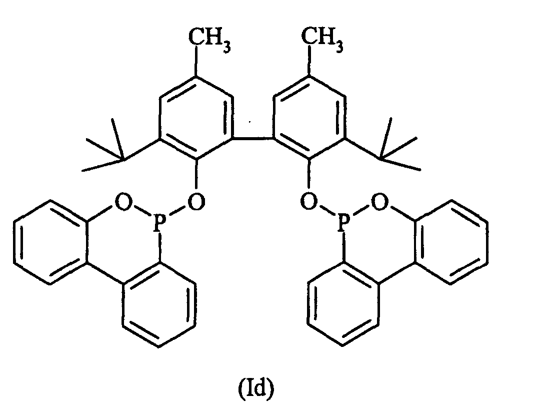

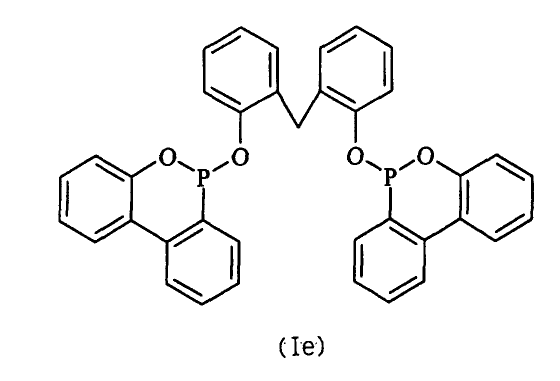

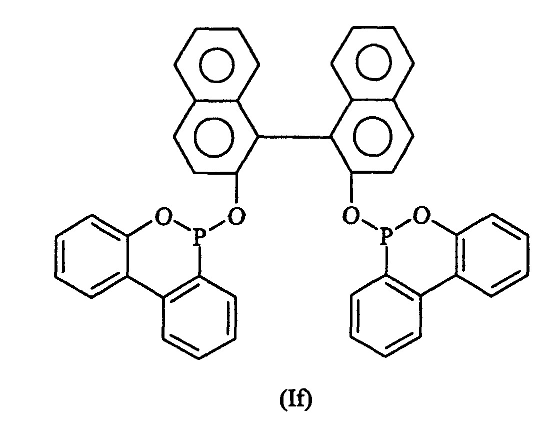

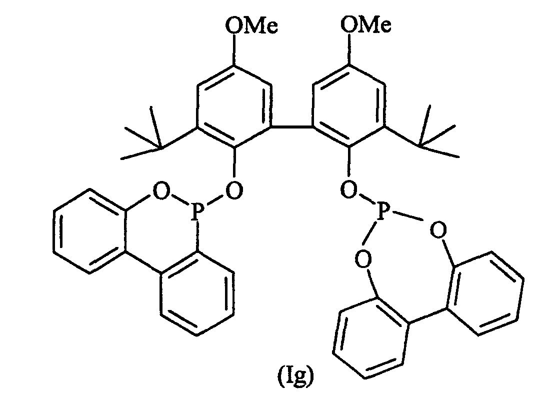

4. Catalyseur selon l'une ou plusieurs des revendications précédentes, qui comporte en outre au moins un autre ligand choisi parmi les halogénures, les amines, les carboxylates, les acétylacétonates, les aryl- ou alkylsulfonates, les hydrures, CO, les oléfines, les diènes, les cyclooléfines, les nitriles, les composés hétérocycliques azotés, les composés aromatiques et hétéroaromatiques, les éthers, PF₃, ainsi que les ligands mono-, bi- ou multidentés de type phosphine, phosphonite et phosphite.

5. Procédé pour l'hydroformylation de composés qui contiennent au moins une double liaison à insaturation éthylénique, par réaction de monoxyde de carbone et d'hydrogène en présence d'un catalyseur d'hydroformylation, **caractérisé en ce qu'**on utilise en tant que catalyseur d'hydroformylation un catalyseur selon l'une des revendications 1 à 4.

6. Procédé selon la revendication 5, **caractérisé en ce que** le catalyseur d'hydroformylation est préparé in situ, ce pour quoi on fait réagir dans les conditions d'une hydroformylation au moins un ligand de type phosphonite de formule générale I tel que défini dans les revendications 1 à 3, un composé ou un complexe d'un métal du VIIIème groupe secondaire et éventuellement un agent d'activation, dans un solvant inerte.

7. Utilisation de catalyseurs comportant un ligand de type phosphonite de formule I, selon l'une des revendications 1 à 3, pour une hydroformylation.
